# EUROPEAN PATENT APPLICATION

(11) **EP 2 404 574 A1**
(43) Date of publication of application: **11.01.2012**
(21) Application number: 11172894.5
(22) Date of filing: 06.07.2011
(51) Int. Cl.: A61F 2/24

(54) **Heart valve prosthesis using different types of living tissue and method of fabricating the same**

(30) Priority: 07.07.2010 KR 20100065306
(71) Applicant: Taewoong Medical Co., Ltd., Kyunggi do 415-873 (KR); Shin, Kyong-Min, Seoul (KR)
(72) Inventor: Shin, Kyong-Min, Seoul (KR); HONG, Kang Sun, Gimpo-si, Gyeonggi-do (KR); JO, Eun Ae, Seoul (KR); KIM, Yong-Jin, Seoul (KR); LEE, Cheul, Seoul (KR); OH, Sam Sea, Seoul (KR); LEE, Chang-Ha, Seoul (KR); LIM, Hong-Gook, Seoul (KR)
(74) Representative: McCartney, Jonathan William

(57) **Abstract**

A heart valve prosthesis and a method of fabricating the same. The heart valve prosthesis uses a leaflet of pig pericardium (10) or a leaflet of cattle pericardium, which has a suitable thickness according to the size of the valve, and a stent. The heart valve prosthesis is fabricated by placing a valve body over a Dacron body (20), binding the valve body to the Dacron body using stitching fiber (30), rolling the Dacron body into a cylindrical form such that the valve body is located inside, binding opposite edges of the Dacron body to each other using stitching fiber, and inserting the resultant structure into a stent (60), which is knitted using a shape memory alloy wire. The heart valve prosthesis is friendly to and is not rejected by human tissue, is free from deformation, and can be implanted using a non-invasive manner without incising the chest wall of a patient.

## Description

### BACKGROUND OF THE INVENTION

### Field of the Invention

The present invention relates, in general, to a heart valve prosthesis using different types of living tissue and to a method of fabricating the same, and more particularly, to a heart valve prosthesis using a pig pericardium or a cattle pericardium and a stent, which is friendly to (or compatible with) and is not rejected by human tissue, is free from deformation, and can be implanted using a non-invasive manner, and a method of fabricating the same.

### Description of the Related Art

Generally, heart valve prostheses are used as a substitute for the human heart valve in terms of their operational method when the heart valve is damaged or malfunctions. Heart valve prostheses are also used in artificial heart or ventricular assist devices (VADs) in order to prevent blood from flowing backwards.

In addition, heart valve prostheses can be divided into their types of a mechanical valve, a tissue valve, and a polymer valve depending on the main components of the materials. The mechanical valve serves to prevent blood from flowing backwards by binding a circular or semicircular hinge to an annular frame in such a fashion that the hinge moves in one direction. The tissue valve is fabricated by shaping a heart valve of an animal or a pericardium of an animal, which surrounds the heart of the animal, into a structure similar to that of the aortic valve or the pulmonary valve. The polymer valve is used by shaping a medical polymer into a structure similar to that of the aortic valve or into a structure that acts as a unidirectional check valve.

Here, in order to prevent blood from flowing backwards, the structure of the aortic valve has three flexible membranes. This type of valve is referred to as a trefoil valve. In the related art, the tissue valve and the polymer valve are fabricated into a similar shape. In addition, leaflets (or cusps) of the heart valve prosthesis are required to have flexibility characteristics similar to those of the leaflets (or cusps) of a heart valve of a human and operate inside the body for a long time without malfunctioning. Therefore, endurance is an important factor in the selection of materials. Furthermore, it is required to select a material having good blood compatibility or use a material to which blood compatibility is imparted.

The fabrication of the trefoil polymer valve includes two steps. First, a part corresponding to the leaflet is fabricated.

This step includes preparing a mold having a shape similar to that of the leaflet, repeatedly immersing the mold into a polymer solution and drying the mold, and then removing a polymer film from the mold. In this fashion, the polymer film having the shape of the leaflet is fabricated. Next, three leaflets are bonded to the inside of a valve conduit, which surrounds the leaflets. It is desirable that the valve conduit has a sinus for each leaflet in order to minimize the area in which blood is congested when the valve is closed, that the thickness of the valve conduit is 2 to 20 times thicker than the leaflet in view of the thickness of the aortic artery inside the human body, and that the valve conduit is sufficiently strong when placed inside the living body.

The process of fabricating the trefoil polymer valve of the related art as described above involves the bonding of the leaflets to the valve conduit, which is separately fabricated by injecting it as a polymer in the form of a solution or molding it using another technique. The time period required for production is several days or longer, it is difficult to expect uniform quality due to the immersion and bonding processes being manually performed, and practicability is low due to low productivity.

Furthermore, since the chest wall of a patient is incised in order to implant the heart valve prosthesis of the related art, it takes a long time for the patient to recover and expenses are increased.

Moreover, while the heart valve prosthesis of the related art is implanted using an artificial valve prosthesis or an artificial tissue prosthesis, it is deformed or deteriorated due to a chemical, physical, or immunological reaction inside the human body, thereby losing functions or becoming non-functional.

### SUMMARY OF THE INVENTION

Accordingly, the present invention has been made keeping in mind of the above problems occurring in the related art, and the present invention is intended to propose a heart valve prosthesis using different types of living tissues and a method of fabricating the same, in which the heart valve prosthesis is fabricated using a novel tissue treatment technique compared to an artificial tissue valve or an artificial tissue prosthesis so that it is neither deformed nor deteriorated by any chemical, physical, or immunological reactions inside a living body.

In addition, the present invention is also intended to propose a heart valve prosthesis using different types of living tissue and a method of fabricating the same, in which an operation can be performed in a non-invasive manner by inserting a heart valve prosthesis body into a stent, which is fabricated by knitting a shape memory alloy, without incising the chest wall of a patient.

Furthermore, the present invention is also intended to propose a heart valve prosthesis that uses different types of living tissue and a method of fabricating the same, in which the heart valve prosthesis can be implanted into a patient having chronic mitral valve insufficiency via the thighbone artery or vein when an incision cannot be performed.

In order to achieve the above object, according to one aspect of the present invention, there is provided a method of fabricating a heart valve prosthesis using different types of living tissues that includes the steps of: forming a valve body by extracting a pericardium leaflet, which surrounds a heart of a pig or cattle, in a rectangular shape and cutting the pericardium leaflet such that three semicircular valves are formed; forming a rectangular piece of Dacron cloth into a Dacron body having three semicircular valve opening spaces, each of the valve opening spaces being smaller than that of a respective semicircular valve of the valve body; placing the valve body over the Dacron body such that the valve body partially overlaps the Dacron body to close the valve opening spaces; stitching the semicircular valves to the Dacron body using stitching fiber; forming a heart valve prosthesis body with the valves connected together by rolling a resultant structure in a cylindrical shape such that the Dacron body connected to the valve body via stitching fiber are exposed to outside and opposite edges of the structure are brought together, and stitching the opposite edges of the structure to each other using stitching fiber; and forming a stent by knitting at least one strand of stent-fabricating wire, such as a hyper elastic shape memory alloy wire, in such a fashion that a number of diamond like spaces are formed, wherein the stent has a hollow cylindrical body, expanding portions at both ends of the body, the expanding portions increasing in diameter with respect to the body, and a number of bent ends formed on terminal ends of the expanding portions, inserting the heart valve prosthesis body into the stent, and connecting the Dacron body and the shape memory alloy wire to each other at several places using stitching fiber.

As set forth above, the heart valve prosthesis is fabricated using a novel tissue treatment technique compared to an artificial tissue valve or an artificial tissue prosthesis so that it is neither deformed nor deteriorated by any chemical, physical, or immunological reactions inside a living body.

In addition, the operation can be performed in a non-invasive manner by inserting a heart valve prosthesis body into a stent, which is fabricated by knitting a shape memory alloy, without incising the chest wall of a patient.

Furthermore, the heart valve prosthesis can be implanted into a patient having chronic mitral valve insufficiency via the thighbone artery or vein when an incision cannot be made.

### BRIEF DESCRIPTION OF THE DRAWINGS

The above and other objects, features and advantages of the present invention will be more clearly understood from the following detailed description when taken in conjunction with the accompanying drawings, in which:
FIG. 1 is a top plan view showing the state in which a pig pericardium and a Dacron body are cut according to the present invention;
FIG. 2 is a top plan view showing the state in which the pig pericardium and the Dacron body have overlapped according to the present invention;
FIG. 3 is a top plan view showing the state in which the pig pericardium and the Dacron body have overlapped each other and have been bound to each other using stitching fiber according to the present invention;
FIG. 4 is a top plan view showing the state in which a heart valve prosthesis body obtained by binding the pig pericardium and the Dacron body to each other using the stitching fiber is rolled into a cylindrical form, which is in turn bound at opposite edges using stitching fiber;
FIG. 5 is a perspective view showing the state in which the heart valve prosthesis body shown in FIG. 4 is inserted into a stent, which has expanding portions on both ends thereof, and bound to the stent using stitching fiber;
FIG. 6 is a perspective view showing the state in which the heart valve prosthesis body shown in FIG. 4 is inserted into a cylindrical stent and bound to the stent using stitching fiber;
FIG. 7 is a perspective view showing the state in which the heart valve prosthesis body shown in FIG. 4 is inserted into a cylindrical stent, which has one expanding portion and one half-expanding portion on both ends thereof, and has been bound to the stent using stitching fiber;
FIG. 8 is a perspective view showing the state in which the heart valve prosthesis body is inserted into a stent having expanding portions at both ends thereof and has been bound to the stent using stitching fiber;
FIG. 9 is a view showing the process of implanting the heart valve prosthesis shown in FIG. 5;
FIG. 10 is a view showing the state in which the heart valve prosthesis shown in FIG. 6 has been implanted in the heart;
FIG. 11 is a view showing the state in which the heart valve prosthesis shown in FIG. 7 is implanted into a heart;

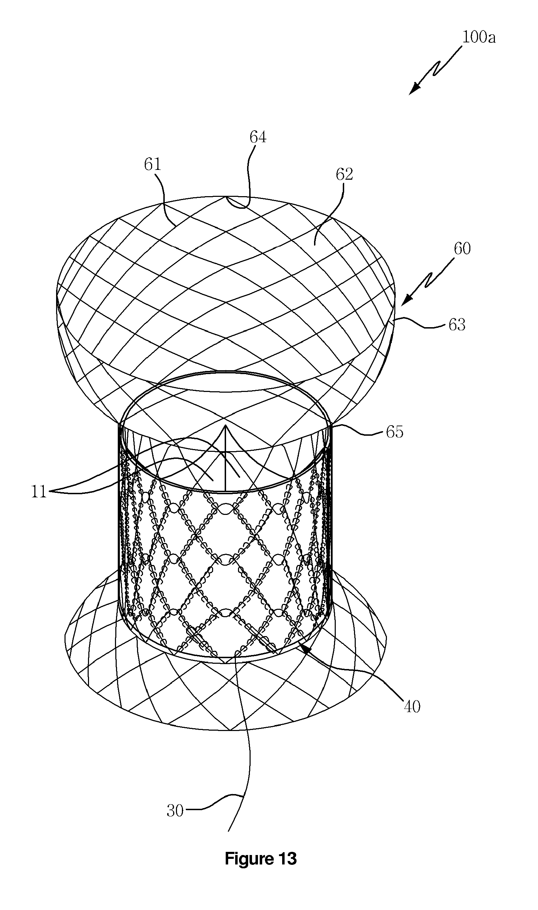

FIG. 12 and 13 are views showing the process of fabricating a heart valve prosthesis by binding a valve body to a Dacron body according to another embodiment of the invention; and
FIG. 14 is an example view showing the heart valve prosthesis of the invention implanted in a heart.

### DETAILED DESCRIPTION OF THE INVENTION

Reference will now be made in greater detail to preferred embodiments of the invention, an example of which is illustrated in the accompanying drawings.

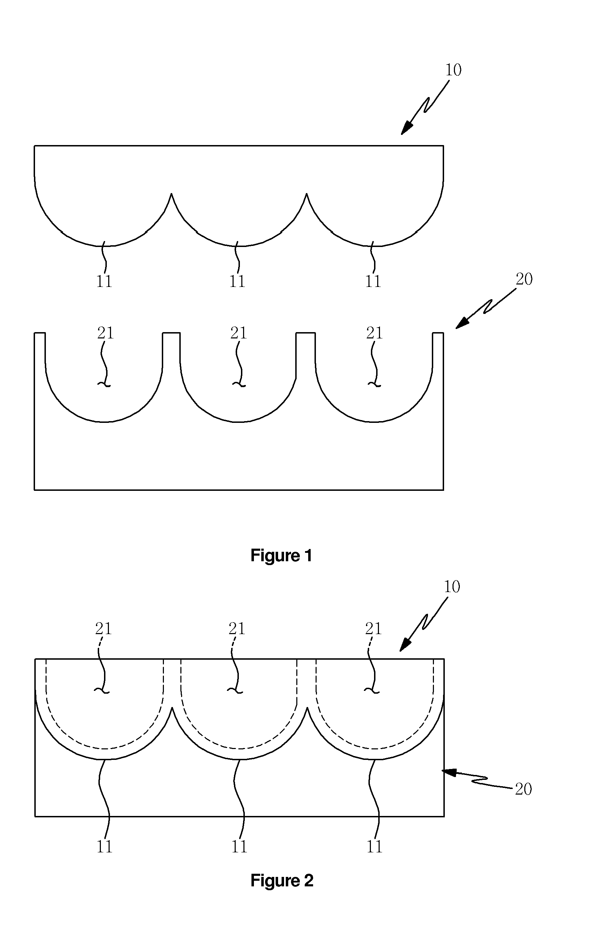

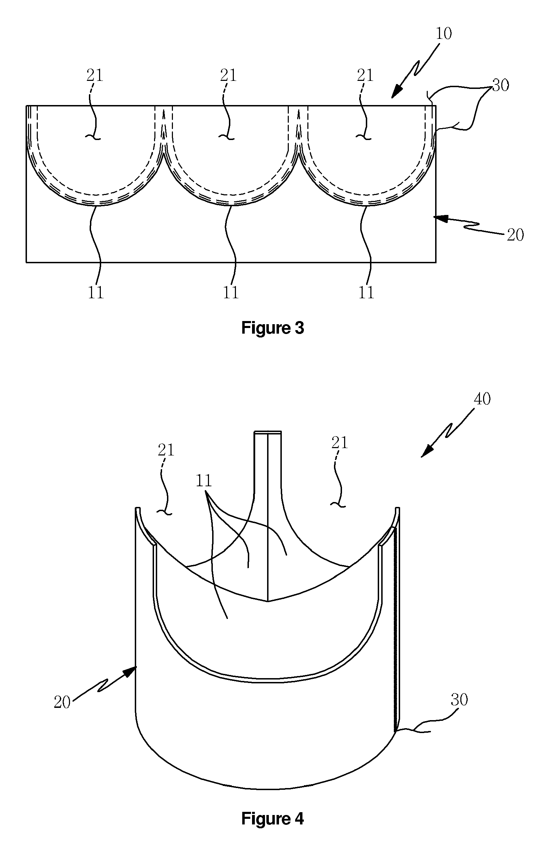

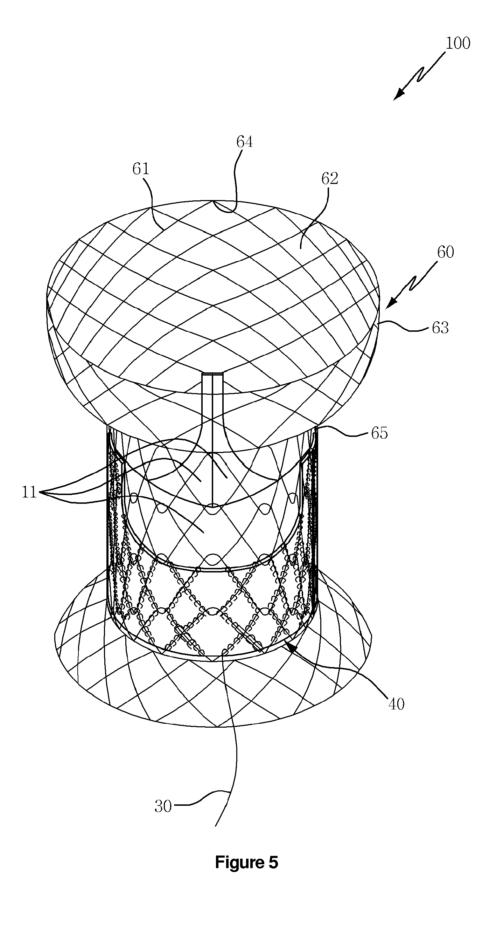

As shown in FIG. 1 to FIG. 5, a method of fabricating a heart valve prosthesis using a pig pericardium according to an embodiment of the present invention fabricates a heart valve prosthesis 100 by placing a valve body 10 over a Dacron body 20, binding the valve body 10 to the Dacron body 20 using stitching fiber 30, rolling the Dacron body 20 into a cylindrical form such that the valve body 10 is located inside, binding opposite edges of the Dacron body 20 to each other using stitching fiber, and inserting the resultant structure into a stent 50, which is knitted using a shape memory alloy wire 51.

First, the valve body 10 is made by extracting a rectangular leaflet from a pig pericardium, which surrounds the heart of a pig, and cutting the pericardium leaflet in such a fashion that three semicircular valves 11 are formed.

Afterwards, a rectangular piece of Dacron cloth is cut in such a fashion that three semicircular valve opening spaces 21 have a smaller area than the valves 11, thereby forming the Dacron body 20.

Subsequently, the valves 11 of the valve body 10 are located to partially overlap the Dacron body 20 such that the valve opening spaces 21 are closed.

Afterwards, the Dacron body 20 and the valves 11 are connected to each other by stitching them along semicircular lines using stitching fiber 30.

The Dacron body 20 connected to the valve body 10 via the stitching fiber 30 is rolled into a cylindrical shape in such a fashion that the Dacron body 20 is exposed to the outside, and opposite edges of the Dacron body 20 are connected to each other by stitching them using the stitching fiber 30. As a result, a heart valve prosthesis body 40 is fabricated.

In addition, the heart valve prosthesis body 40 is inserted into a stent 60. The stent 60 is fabricated by knitting at least one strand of stent-fabricating wire 61 made of, for example, a hyper elastic shape memory alloy in such a fashion that a number of diamond like spaces 62 are formed. The stent 60 has a hollow cylindrical body 65 and expanding portions 63 at both ends of the body 65, the expanding portions 63 increasing in diameter with respect to the body 65. A number of bent ends 64 are formed on terminal ends of the expanding portions 63. Afterwards, the Dacron body 20 and the shape memory alloy wire 61 are connected to each other at several places using the stitching fiber 30.

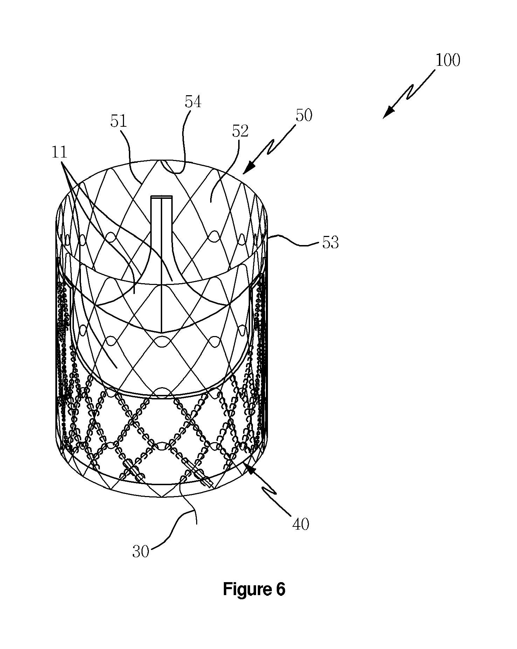

As shown in FIG. 6, in another embodiment, a stent 50 may be fabricated by knitting or crossing at least one strand of shape memory alloy wire 51 in a zigzag pattern in such a fashion that a number of spaces 52 are formed in a cylindrical body 53 thereof. A number of bent ends 54 are also formed along the circumference of both ends of the cylindrical body 53.

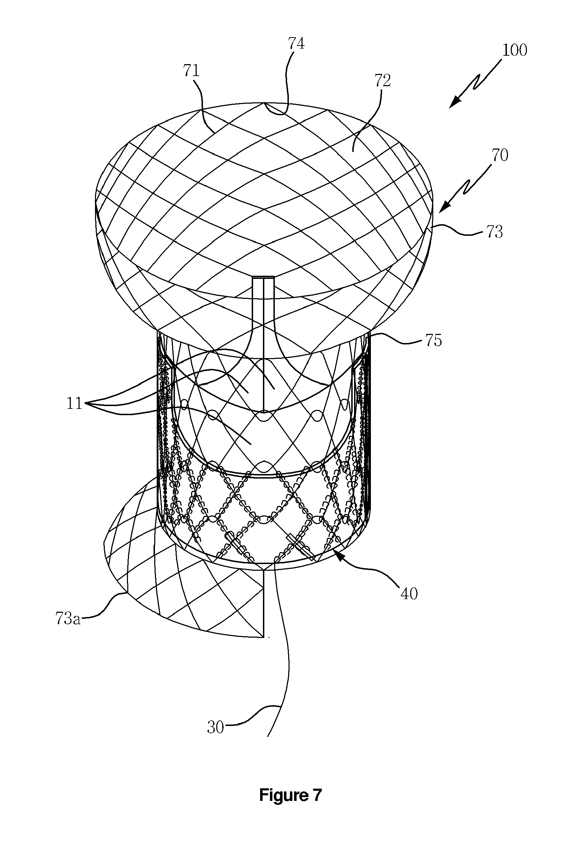

As shown in FIG. 7, in a further embodiment, a stent 70 may be fabricated by knitting at least one strand of stent-fabricating wire 61 made of, for example, a hyper elastic shape memory alloy in such a fashion that a number of diamond like spaces 62 are formed. The stent 70 has a hollow cylindrical body 75, an expanding portion 73 on one end of the body 75, and a half-expanding portion 73a on the other end of the body 75. A number of bent ends 74 are also formed along the circumference of both ends of the body 75.

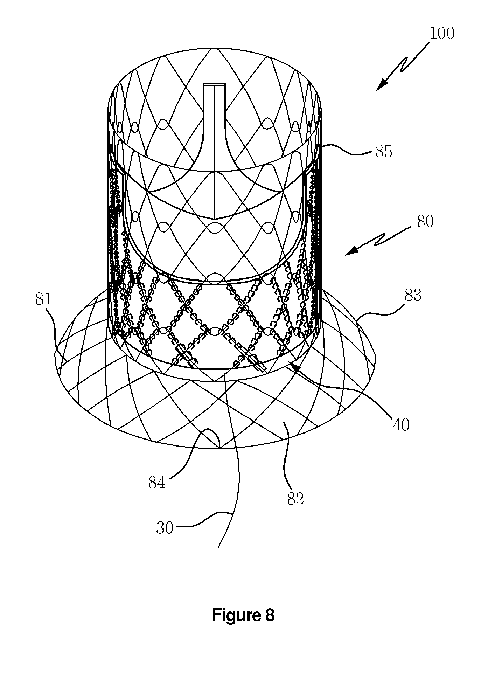

As shown in FIG. 8, in still another embodiment, a stent 80 may be fabricated by knitting at least one strand of stent-fabricating wire 61 made of, for example, a hyper elastic shape memory alloy in such a fashion that a number of diamond like spaces 62 are formed.

The stent 80 has a hollow cylindrical body 85 and an expanding portion 83, which expands in diameter, on one end of the body 85. A number of bent ends 84 are also formed along the circumference of both ends of the body 85.

It is possible to perform an operation by inserting the stent into the pulmonary artery 150.

The operation using the heart valve prosthesis 100 of the present invention as configured above can be performed as follows.

First, the heart valve prosthesis 100 is reduced in volume and inserted into a catheter, which is a general type of a stent inserting device.

In this case, the heart valve prosthesis 100 is inserted into an outer tube of the catheter, and is exposed to the outside via a movable tube that moves back and forth inside the outer tube.

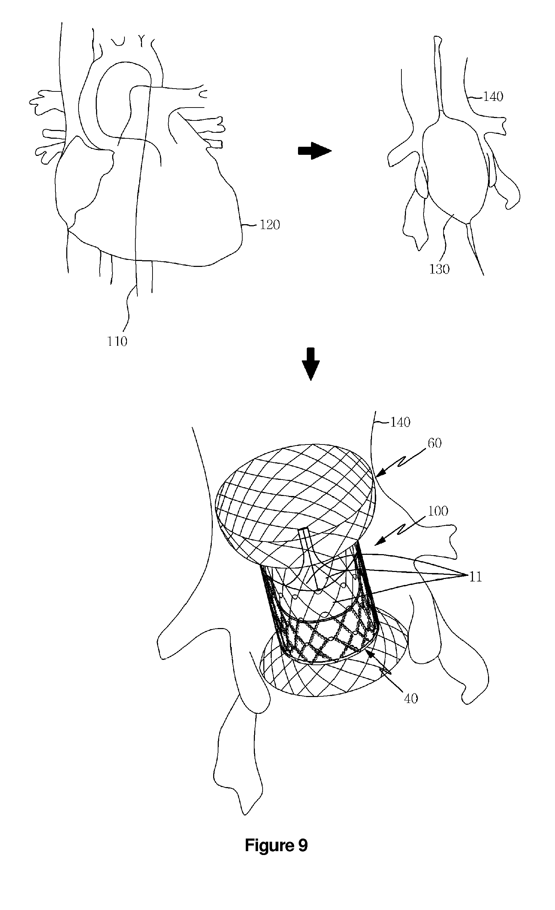

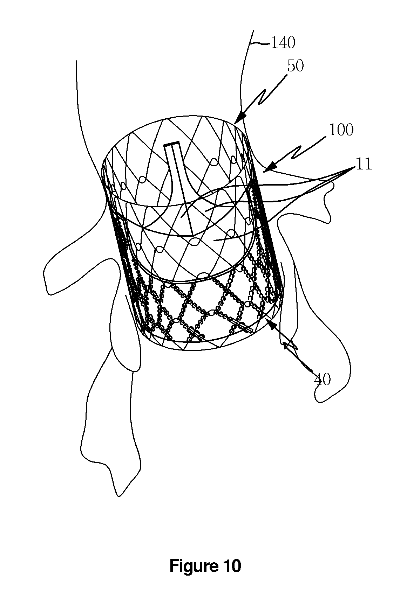

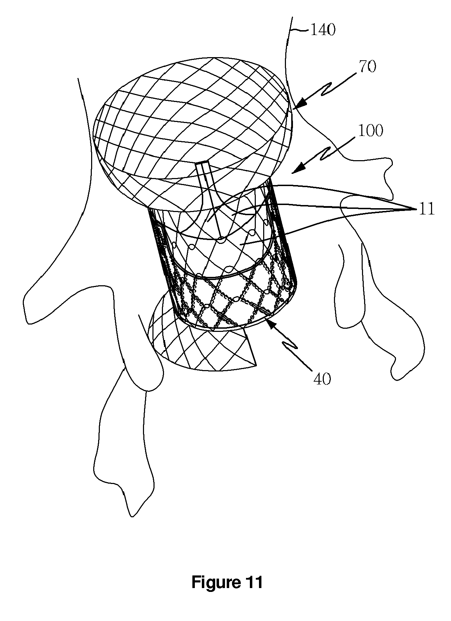

As shown in FIG. 9 to FIG. 11, in order to implant the heart valve prosthesis 100, a guide wire 110, which can introduce a catheter, is first inserted into the heart 120 through the thighbone artery or vein. The heart valve prosthesis 100 is then located inside the main artery that is operated on.

Although the operation of implanting the heart valve prosthesis 100 inside the aortic artery 140 is described by way of an example, the heart valve prosthesis 100 can also be implanted inside the pulmonary artery 150. Therefore, the heart valve prosthesis 100 can be used for both the aortic artery 140 and the pulmonary artery 150, and the applicability thereof is increased.

Here, when the aortic artery 140 or pulmonary artery 150 needs to be expanded, an operation can be performed after the artery is expanded using a balloon 130, that is, a surgical device having the form of a balloon.

Afterwards, a catheter is inserted along the guide wire 110 and is located inside the aortic artery 140 or pulmonary artery 150. In this state, pushing forward the movable tube extracts the heart valve prosthesis 100 from the outer tube.

The heart valve prosthesis 100 extracted from the outer tube restores the cylindrical shape due to the restoring function of the shape memory wire 51, 61, 71 of the stent 50, 60, 70, while the stent 50, 60, 70 presses and supports the inner wall of the aortic artery 140 or pulmonary artery 150.

Here, the aortic artery 140 is blocked by the valves 11 of the valve body 10 such that the blood flows from the lower part to the upper part but does not flow from the upper part to the lower part of the aortic artery 140.

In other words, when the blood flows from the lower part to the upper part of the aortic artery 140, the valves 11, which are in contact with each other, are spread by the flowing force of the blood. In contrast, when the blood flows from the upper part to the lower part of the aortic artery 140, the valves 11 are brought into close contact with each other, thereby preventing the blood from flowing.

In addition, the stent 70 has the half-expanding portion 73a on one end thereof, which is increased halfway in diameter. When the stent 70 is inserted into the aortic artery 140, the half-expanding portion 73a is expanded halfway in order to prevent it from coming into contact with the mitral valve.

The heart valve prosthesis 100 is fabricated using a novel tissue treatment technique in which the valve body 10 is made of a leaflet of pig pericardium or a leaflet of cattle pericardium having a suitable thickness according to the size of the valve, and the Dacron body 20 is made of polyester. Consequently, even when implanted into a living body, the properties of the heart valve prosthesis 100 do not change and are not deteriorated by chemical, physical, or immunological reactions.

Accordingly, there is an advantage in that the heart valve prosthesis 100 neither malfunctions nor becomes non-functional. In addition, an operation can be performed without incising the chest wall of a patient, thereby reducing the burden on the patient.

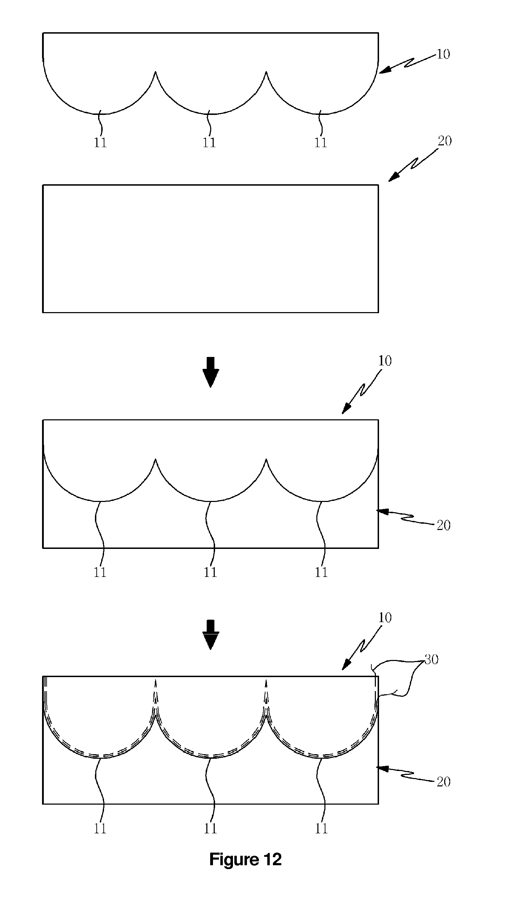

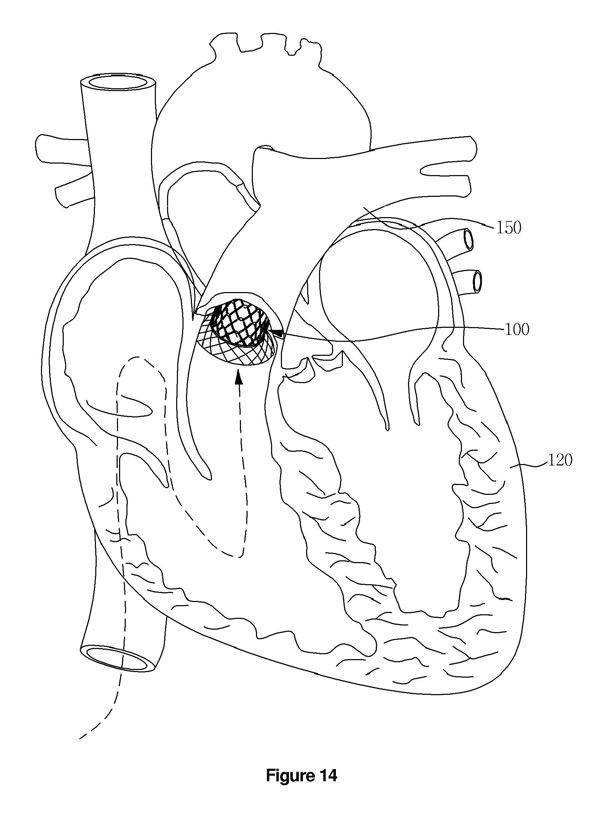

As shown in FIG. 12 and FIG. 14, in yet another embodiment, a heart valve prosthesis 100a may be provided. In the heart valve prosthesis 100a, the Dacron body 20 can be configured as a rectangular plate, which can be bound to the valve body 10.

That is, the valves 11 of the valve body 10 are stitched to one side of the Dacron body 20 into the form of a semicircle using the stitching fiber 30, the Dacron body 20 is rolled into the form of a cylinder, and then the opposite edges of the cylinder are bound to each other using the stitching fiber 30. The resultant structure is subsequently inserted into the stent 50, 60, 70, which is then connected to the Dacron body 20 using the stitching fiber 30.

Here, the heart valve prosthesis 100a can be implanted into the pulmonary artery 150 using the catheter. The heart valve prosthesis 100a may be used exclusively for the pulmonary artery 150.

Furthermore, although the heart valve prostheses 100 and 100a have been described as being implanted into the heart 120 without incising the chest wall, the heart valve prostheses 100 and 100a can be implanted in the state in which the chest wall is incised if necessary, without the use of the catheter.

Although the exemplary embodiments of the present invention have been described for illustrative purposes, those skilled in the art will appreciate that various modifications, additions and substitutions are possible, without departing from the scope and spirit of the invention as disclosed in the accompanying claims.

## Claims

1. A method of fabricating a heart valve prosthesis using different types of living tissues, comprising:
forming a valve body by extracting a pericardium leaflet, which surrounds a heart of a pig or cattle, in a rectangular shape and cutting the pericardium leaflet such that three semicircular valves are formed;
forming a rectangular piece of Dacron cloth into a Dacron body having three semicircular valve opening spaces, each of the valve opening spaces being smaller than that of a respective semicircular valve of the valve body;
placing the valve body over the Dacron body such that the valve body partially overlaps the Dacron body to close the valve opening spaces;
stitching the semicircular valves to the Dacron body using stitching fiber;
forming a heart valve prosthesis body with the valves connected together by rolling a resultant structure in a cylindrical shape such that the Dacron body connected to the valve body via stitching fiber are exposed to outside and opposite edges of the structure are brought together, and stitching the opposite edges of the structure to each other using stitching fiber; and
forming a stent by knitting at least one strand of stent-fabricating wire, such as a hyper elastic shape memory alloy wire, in such a fashion that a number of diamond like spaces are formed, wherein the stent has a hollow cylindrical body, expanding portions at both ends of the body, the expanding portions increasing in diameter with respect to the body, and a number of bent ends formed on terminal ends of the expanding portions, inserting the heart valve prosthesis body into the stent, and connecting the Dacron body and the shape memory alloy wire to each other at several places using stitching fiber.

2. The method of claim 1, wherein the stent is formed by knitting or crossing at least one strand of shape memory alloy wire in a zigzag pattern in such a fashion that a number of spaces are formed in a cylindrical body thereof, wherein the stent has a cylindrical body and a number of bent ends are formed along a circumference of both ends of the cylindrical body.

3. The method of claim 1, wherein the stent is formed by knitting at least one strand of stent-fabricating wire, such as a hyper elastic shape memory alloy, in such a fashion that a number of diamond like spaces are formed, wherein the stent has a hollow cylindrical body, an expanding portion on one end of the body, a half-expanding portion on the other end of the body, and a number of bent ends formed along a circumference of both ends of the body.

4. The method of any of claims 1-3, wherein the Dacron body is configured as a rectangular plate, which is bound to the valve body.

5. The method of claim 1, wherein the stent is formed by knitting at least one strand of stent-fabricating wire, such as a hyper elastic shape memory alloy, in such a fashion that a number of diamond like spaces are formed, wherein the stent has a hollow cylindrical body, an expanding portion on one end of the body, the expanding portion expanding in diameter, and a number of bent ends formed along a circumference of both ends of the body.

6. A heart valve prosthesis using different types of living tissue, comprising:
a heart valve prosthesis body comprising:
a valve body made of a pericardium leaflet, which surrounds a heart of a pig or cattle, the pericardium leaflet being extracted in a rectangular shape according to a size of a valve and cut such that three semicircular valves are formed, and
a Dacron body made of a rectangular piece of Dacron cloth, wherein the Dacron body has three semicircular valve opening spaces, each of the valve opening spaces being smaller than that of a respective semicircular valve of the valve body, and
wherein the valves of the valve body are located to partially overlap the Dacron body such that the valve opening spaces are closed, the Dacron body and the valves are connected to each other by stitching them along semicircular lines using stitching fiber, the Dacron body connected to the valve body via stitching fiber is rolled into a cylindrical shape in such a fashion that the Dacron body is exposed to outside, and both ends of the Dacron body are connected to each other by stitching them using the stitching fiber; and
a stent having a cylindrical body and a number of bent ends formed along a circumference of both ends of the cylindrical body, wherein the stent is made of at least one strand of shape memory alloy wire, which is knitted or crossed in a zigzag pattern in such a fashion that a number of spaces are formed in the cylindrical body,
wherein the heart valve prosthesis body is inserted into the stent, and the Dacron body and the shape memory alloy wire are connected to each other at several places using stitching fiber.

7. The heart valve prosthesis of claim 6, wherein the stent is formed by knitting at least one strand of stent-fabricating wire, such as hyper elastic shape memory alloy, in such a fashion that a number of diamond like spaces are formed, wherein the stent has a hollow cylindrical body, expanding portions at both ends of the body, the expanding portions increasing in diameter with respect to the body, and a number of bent ends formed on terminal ends of the expanding portions.

8. The heart valve prosthesis of claim 6, wherein the stent is formed by knitting at least one strand of stent-fabricating wire, such as a hyper elastic shape memory alloy, in such a fashion that a number of diamond like spaces are formed, wherein the stent has a hollow cylindrical body, an expanding portion on one end of the body, a half-expanding portion on the other end of the body, and a number of bent ends formed along a circumference of both ends of the body.

9. The heart valve prosthesis of any of claims 6-8, wherein the Dacron body is configured as a rectangular plate, which is bound to the valve body.

10. The heart valve prosthesis of claim 6, wherein the stent is formed by knitting at least one strand of stent-fabricating wire, such as a hyper elastic shape memory alloy, in such a fashion that a number of diamond like spaces are formed, wherein the stent has a hollow cylindrical body, an expanding portion on one end of the body, the expanding portion expanding in diameter, and a number of bent ends formed along a circumference of both ends of the body.
